# FASCICULE DE BREVET EUROPEEN

(11) **EP 0 757 545 B1**
(45) Date de publication et mention de la délivrance du brevet: **18.07.2001**
(21) Numéro de dépôt: 95918643.8
(22) Date de dépôt: 26.04.1995
(51) Int. Cl.: A61B 17/16, A61B 17/56

(54) **MATERIEL POUR LA CORRECTION DE DEFORMATIONS DES OS LONGS**
VORRICHTUNG ZUR KORREKTION VON VERBIEGUNGEN DER LANGEN KNOCHEN
APPARATUS FOR CORRECTING LONG BONE DEFORMATION

(30) Priorité: 27.04.1994 FR 9405332
(43) Date de publication de la demande: 12.02.1997
(73) Titulaire: Mortier, Jean-Pierre, 75005 Paris (FR)
(72) Inventeur: Mortier, Jean-Pierre, 75005 Paris (FR)
(74) Mandataire: Poncet, Jean-François
(86) Numéro de dépôt international: FR9500544
(87) Numéro de publication internationale: WO9528887

(56) Documents cités:
- EP-A- 0 552 109
- WO-A-94/02073
- DE-A- 3 724 479
- FR-A- 2 362 616
- FR-A- 2 456 506
- FR-A- 2 676 353
- FR-A- 2 686 012
- GB-A- 2 126 903
- US-A- 5 197 966

## Description

La présente invention concerne un matériel destiné à corriger les déformations de certains os longs en particulier les petits os longs du corps humain. Le matériel selon l'invention permet de corriger notamment les déformations du premier rayon du pied, principalement en Hallux Valgus.

Le premier rayon du pied comprend trois os longs successifs articulés les uns aux autres, à savoir le premier métatarsien, la première phalange et la seconde phalange. Une déformation en Hallux Valgus est une angulation excessive du premier rayon du pied, à sommet interne.

On peut être tenté de corriger cette angulation excessive en agissant par ostéotomie, d'ouverture interne ou de fermeture externe, sur le premier métatarsien. Malheureusement, jusqu'à ce jour, aucune correction angulaire précise n'a été possible, faute d'instruments de synthèse spécifiques adaptés.

Ainsi, on connaît notamment une agrafe-plaque d'ostéosynthèse pour tête de tibia ou de fémur, décrite dans le document WO-A-94 02073. Ce dispositif comprend un corps rigide, a priori non déformable, courbé en S et solidaire à ses deux extrémités de deux broches divergentes à dents provoquant une compression des fragments osseux de part et d'autre du trait de fracture. Outre le fait qu'un tel dispositif n'est pas adapté à une pénétration dans l'os cortical, pénétration qui est nécessaire pour la correction des déformations du pied, et qu'il n'exerce par sa forme elle-même aucune action de modification angulaire, la correction éventuellement obtenue est indépendante de la forme du corps rigide et ne dépend que de l'angle de l'ostéotomie angulaire pratiquée avant pénétration des broches. Sachant qu'il est toujours difficile de réaliser un angle d'ostéotomie précis, le dispositif ne permet donc pas de réaliser aisément une correction angulaire précise. Egalement, le dispositif assure une compression des fragments osseux, de sorte qu'il n'est pas adapté au maintien d'une ostéotomie d'ouverture interne.

On connaît également, du document FR-A-2 686 012, une lame-plaque adaptée pour la correction angulaire du tibia. Par la forme en S du corps, et par la présence d'une lame, le dispositif est adapté à la seule correction du tibia, et n'est pas approprié pour les autres os tels que les os longs du pied. En outre, le corps étant très rigide pour supporter les contraintes mécaniques importantes du tibia, il n'est pas déformable et présente un angle fixe entre la lame et le corps. La correction angulaire de l'ostéotomie est donc réglée en variant lors de la pose l'angle de pénétration de la lame dans l'os épiphysaire, et en ramenant progressivement la portion diaphysaire de l'os au contact du corps. Un tel réglage est délicat et malaisé. En outre, en fonction de la correction angulaire réalisée, il se produit alors un écart entre le corps et la surface externe de la portion épiphysaire de l'os. Et le dispositif n'est pas adapté au traitement des petits os longs car la distance entre l'orifice de pénétration de la lame et le trait d'ostéotomie est alors trop faible pour admettre des variations de l'angle de pénétration. Dès qu'on voudrait choisir un angle de pénétration aigu, la lame rencontrerait en effet le trait d'ostéotomie.

Le document EP-A-0 552 109 décrit une agrafe pour épingler un ligament sur un os. Un tel dispositif ne permet pas de corrections angulaires, et la proximité des broches et des doigts de calage le rend impropre au maintien d'une ostéotomie.

Le document FR-A-2 362 616 décrit une plaque-agrafe à conformer en tube, pour le maintien rectiligne d'un os. Ce dispositif ne permet pas de corrections angulaires.

Le problème proposé par la présente invention est ainsi de définir un matériel adapté pour la correction aisée et efficace des déformations osseuses des petits os longs, notamment des déformations du premier rayon du pied en Hallux Valgus. Le matériel doit assurer par lui-même la correction angulaire précise, sans qu'il soit besoin de respecter une grande précision dans l'ostéotomie à réaliser. Et le matériel doit ensuite assurer un maintien angulaire correct des portions successives d'os après ostéotomie, sans fragiliser l'os, en permettant notamment de maximiser la distance entre l'ostéotomie et les éléments d'ancrage pénétrant dans l'os.

Le matériel selon l'invention doit également être utilisable pour la correction de déformations associées ou non dans le plan vertical, telles que l'abaissement ou l'élévation du métatarsien, ou dans le plan rotatoire affectant la supination ou la pronation, ou dans la longueur par raccourcissement ou allongement. Il peut être également utilisé pour la correction d'autres métatarsiens, particulièrement lors des troubles de rotation du cinquième métatarsien, ou pour la correction d'autres petits os longs tels que les os de la main.

Pour atteindre ces objets ainsi que d'autres, le matériel selon l'invention pour la correction de déformations osseuses des os longs comprend au moins un dispositif de maintien de l'os comportant :
- un corps allongé rigide comportant deux segments principaux rectilignes solidaires et se raccordant angulairement l'un à l'autre par une zone de raccordement angulaire selon un angle (A) adaptable pour être sensiblement égal à la correction angulaire à réaliser sur un os, ledit corps étant en un matériau et selon une forme admettant sans rupture et conservant les déformations angulaires permanentes dans la zone de raccordement angulaire,
- des premiers moyens d'ancrage, prévus sur le premier segment principal du corps pour se fixer transversalement dans l'os selon une direction faisant un angle (B) déterminé tel qu'un angle droit avec l'axe longitudinal de l'os, les premiers moyens d'ancrage comprenant au moins une broche se développant selon une direction faisant généralement ledit angle déterminé (B) à l'axe longitudinal du premier segment principal de corps,
- des seconds moyens d'ancrage, prévus sur le second segment principal du corps et adaptés pour se fixer transversalement dans l'os au cours d'une étape ultérieure, après fixation des premiers moyens d'ancrage, de sorte que, après ostéotomie d'ouverture ou de fermeture réalisée entre deux portions adjacentes de l'os selon un angle sensiblement égal à la correction à obtenir, et après ancrage des premiers moyens d'ancrage du dispositif selon l'angle déterminé (B) dans l'os en plaçant la zone de raccordement angulaire en regard de la zone d'ostéotomie, le dispositif détermine le décalage angulaire entre les deux portions adjacentes d'os, l'ancrage ultérieur des seconds moyens d'ancrage permettant de fixer le décalage angulaire.

Selon un premier mode de réalisation :
- les seconds moyens d'ancrage comprennent au moins un trou d'adaptation de vis à os,
- les moyens d'ancrage sont adaptés pour pénétrer dans l'os en faisant saillie sur la face concave du corps.

Un tel dispositif comprenant deux broches de segment principal de corps est alors une plaque-agrafe adaptée à la correction de déformations du premier métatarsien.

Un tel dispositif comprenant une seule broche de segment principal de corps est alors une plaque-agrafe adaptée à la correction de déformations du cinquième métatarsien.

Selon l'invention, on prévoit un dispositif complémentaire ayant un corps allongé rigide composé d'un premier segment principal rectiligne et d'un second segment principal rectiligne se raccordant l'un à l'autre par une zone de raccordement angulaire et dans lequel :
- des premiers moyens d'ancrage comprennent un premier groupe de deux broches décalées longitudinalement sur le corps,
- des seconds moyens d'ancrage comprennent un second groupe de deux broches décalées longitudinalement sur le corps,
- les broches font saillie sur la face convexe du corps.

Ce dispositif complémentaire est alors une agrafe adaptée à la correction de déformations de la première phalange.

La combinaison des deux dispositifs permet la correction du premier métatarsien et de la première phalange.

D'autres objets, caractéristiques et avantages de la présente invention ressortiront de la description suivante de modes de réalisation particuliers, faite en relation avec les figures jointes, parmi lesquelles :
- la figure 1 illustre, en vue de dessus, le squelette d'un pied présentant une déformation de type Hallux Valgus ;
- la figure 2 illustre, en vue de dessus, le squelette du pied de la figure 1 après correction du premier rayon du pied au moyen du matériel selon la présente invention ; la figure illustre également la correction du cinquième métatarsien ;
- la figure 3 est une vue en perspective d'un dispositif selon un mode de réalisation de la présente invention ;
- la figure 4 illustre en perspective une variante du mode de réalisation du dispositif de la figure 3 ;
- la figure 5 illustre en perspective une seconde variante du dispositif de la figure 3, adaptée pour la correction du cinquième métatarsien ;
- la figure 6 est une vue en perspective d'un dispositif complémentaire selon un mode de réalisation de l'invention ;
- la figure 7 est une vue de dessus d'un guide de perçage selon l'invention, adapté pour réaliser les tunnels forés dans l'os pour l'engagement des premiers moyens d'ancrage ;
- la figure 8 est une vue de côté du guide de perçage de la figure 7 ;
- la figure 9 est une vue de face du guide de perçage de la figure 7 ;
- la figure 10 est une vue de côté d'un rapporteur selon l'invention, utilisé en per-opératoire pour mesurer l'angle de correction angulaire désiré sur l'os ;
- la figure 11 est une vue de dessus du rapporteur de la figure 10 ;
- la figure 12 est une vue de côté d'un premier élément de dispositif cintreur selon l'invention, pour donner à la plaque-agrafe un angle conforme à la correction angulaire à réaliser sur l'os ;
- la figure 13 est une vue de dessus du premier élément de dispositif cintreur de la figure 12 ; et
- la figure 14 est une vue de côté du second élément de dispositif cintreur, utilisé en association avec le premier élément de la figure 12 pour donner à une plaque-agrafe un angle de correction désiré.

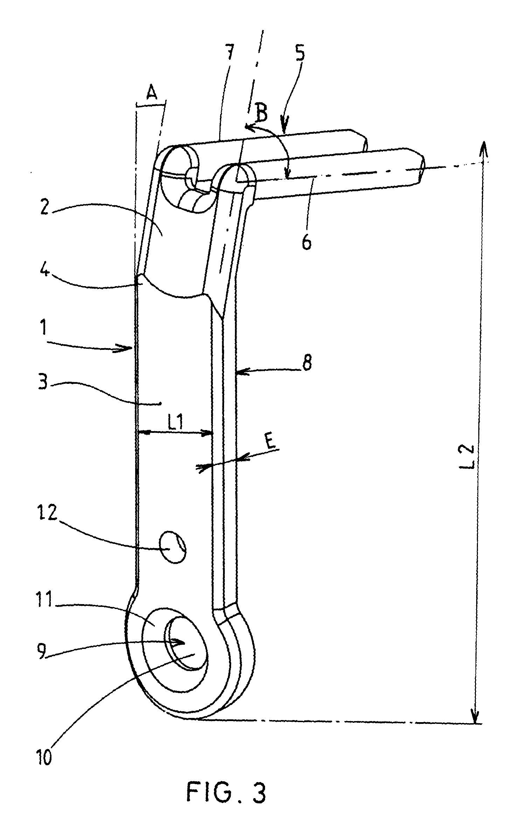

Dans le mode de réalisation illustré sur la figure 3, le dispositif selon l'invention comprend un corps 1 allongé rigide, comportant un premier segment principal 2 rectiligne et un second segment principal 3 rectiligne solidaires l'un de l'autre et se raccordant angulairement l'un à l'autre par une zone de raccordement angulaire 4 selon un angle A choisi égal à la correction angulaire à réaliser sur un os.

Des premiers moyens d'ancrage 5 sont prévus sur le premier segment principal 2 du corps 1, pour se fixer transversalement dans l'os. Dans le mode de réalisation illustré, les premiers moyens d'ancrage 5 comprennent deux broches 6 et 7, solidaires du corps 1, parallèles l'une à l'autre et généralement orientées selon un angle B déterminé tel qu'un angle droit par rapport à la direction longitudinale du premier segment principal 2, en faisant saillie sur la face concave 8 du corps 1. Les deux broches 6 et 7 parallèles sont décalées latéralement l'une par rapport à l'autre et se développent selon un plan faisant généralement l'angle déterminé B tel qu'un angle droit par rapport à l'axe longitudinal du premier segment principal 2 de corps 1. Les broches 6 et 7 peuvent ainsi se fixer dans l'os au cours d'une première étape de fixation.

Des seconds moyens d'ancrage 9 sont prévus sur le second segment principal 3 du corps 1, pour se fixer transversalement dans l'os au cours d'une étape ultérieure, après fixation des premiers moyens d'ancrage 5. Dans le mode de réalisation illustré sur la figure, les seconds moyens d'ancrage 9 comprennent un trou 10 pour l'adaptation d'une tête de vis à os dont la tige est destinée à faire saillie sur la face concave 8 du corps 1. Le trou 10 présente par exemple une portion 11 en couronne sphérique, pour l'adaptation d'une tête à portée sphérique de vis à os.

En outre, le corps 1 peut avantageusement comprendre, au voisinage du trou 10 d'adaptation de vis à os, un trou secondaire 12 pour le passage d'une broche amovible permettant une fixation provisoire ou un renforcement de la fixation du dispositif sur l'os.

L'angle A peut avantageusement être égal à 15° environ. Toutefois, cet angle peut être différent, en fonction de la correction à obtenir sur le métatarsus verus.

Dans tous les modes de réalisation, on prévoit avantageusement la possibilité de déformer le corps 1 dans sa zone de raccordement angulaire 4 pour modifier l'angle A et pour l'adapter à la correction d'orientation verticale à réaliser, éventuellement associée à une correction latérale. Pour cela, on réalise le corps 1 en un matériau admettant sans rupture et conservant les déformations angulaires permanentes dans sa zone de raccordement angulaire 4. On peut utiliser un acier inoxydable biologiquement compatible. On donne en outre au corps une forme permettant les déformations angulaires permanentes : dans la zone de raccordement angulaire 4, le corps présente une épaisseur E sensiblement inférieure à sa largeur L1.

Le corps 1, dans ce mode de réalisation de la figure 3, présente la forme d'une plaque allongée, dont la largeur L1 est sensiblement plus grande que l'épaisseur E. Sa longueur L2 est comprise entre le tiers et la moitié de la longueur de l'os qu'il est destiné à corriger. Pour une application à la correction d'un premier métatarsien, la longueur L2 est avantageusement comprise entre 2 et 3 cm environ.

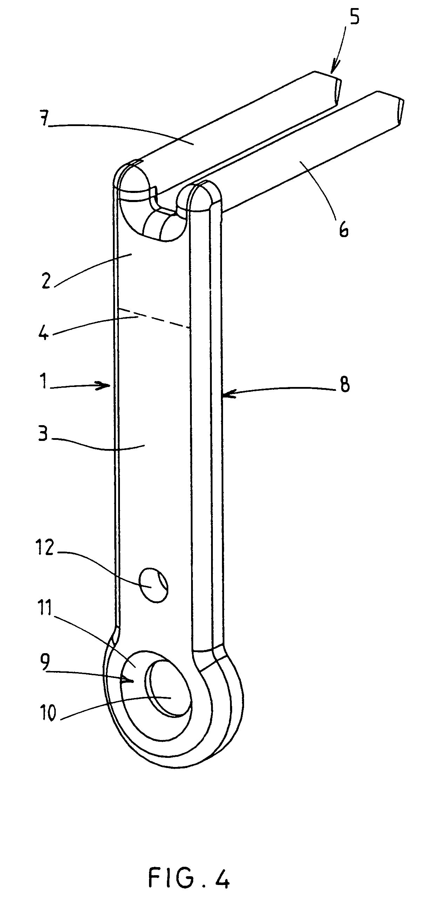

Dans la variante illustrée sur la figure 4, le dispositif comprend les mêmes éléments que dans le mode de réalisation de la figure 3, et ces éléments communs sont repérés par les mêmes références numériques.

Toutefois, dans ce mode de réalisation, le corps 1 est une plaque rectiligne, et sa zone de raccordement angulaire 4 reste à former selon un angle approprié par un dispositif de cintrage, en fonction de la correction angulaire de l'os à obtenir.

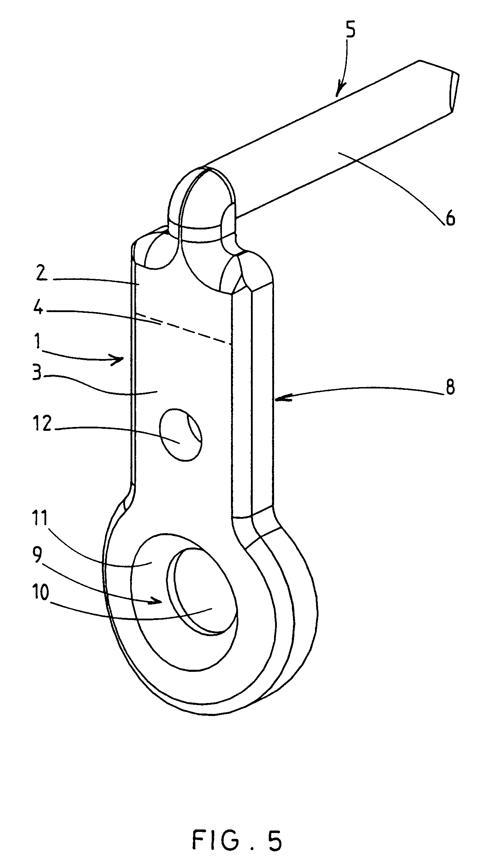

Dans la variante illustrée sur la figure 5, le dispositif comprend également des moyens similaires de ceux du mode de réalisation de la figure 3, et ces moyens similaires sont repérés par les mêmes références numériques.

Dans cette variante de la figure 5, le corps 1 est également une plaque rectiligne, et la zone de raccordement angulaire 4 reste à former selon un angle approprié par un dispositif de cintrage.

En outre, dans cette variante, les premiers moyens d'ancrage 5 comprennent une broche unique 6. On réalise ainsi un dispositif adapté notamment à la correction du cinquième métatarsien.

La ou les broches des premiers moyens d'ancrage 5 font avec le premier segment 2 du corps 1 un angle B déterminé. L'invention prévoit un guide de perçage permettant de forer dans l'os des trous de pénétration faisant le même angle B par rapport à la direction longitudinale de l'os.

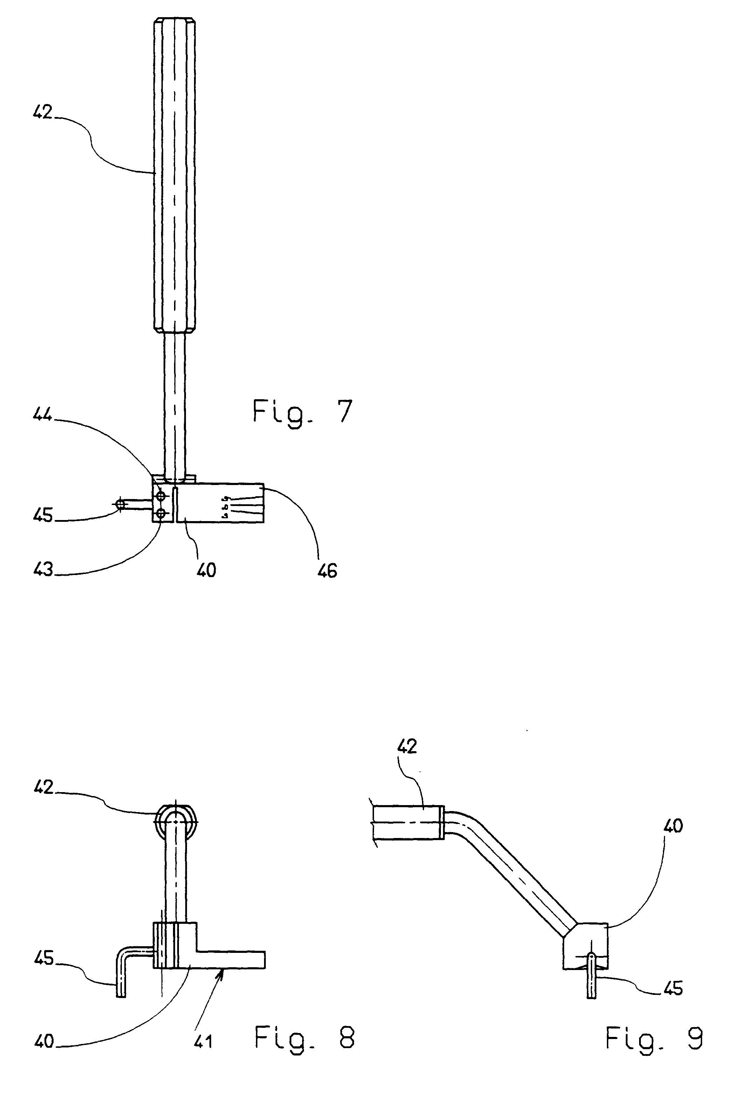

On utilise pour cela un guide de perçage tel qu'illustré sur les figures 7 à 9, comprenant un corps 40 à face inférieure d'appui 41 plane, tenu par un manche 42 transversal, et muni de deux trous traversants 43 et 44 perpendiculaires à la face inférieure d'appui 41. Dans ce mode de réalisation, l'angle B est un angle droit. En alternative, on peut incliner les trous 43 et 44 pour réaliser un angle B différent de 90°. Un crochet longitudinal 45 permet de caler le positionnement du dispositif de perçage par rapport à une extrémité d'os. Une graduation 46, en extrémité du corps 40, permet un réglage d'orientation latérale des parties d'os de part et d'autre d'une ostéotomie, si nécessaire.

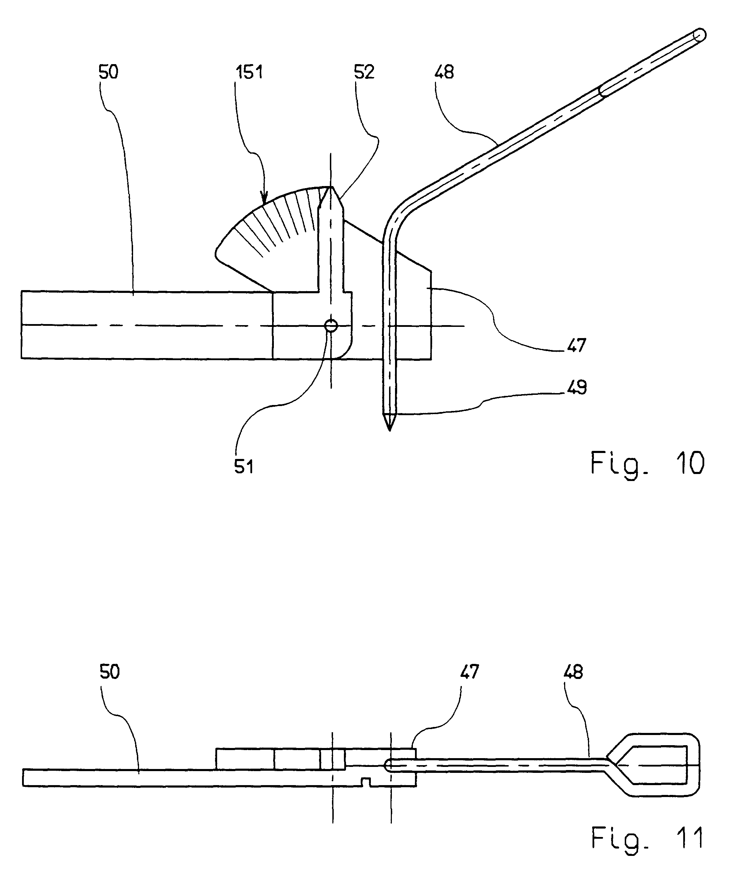

Lorsque l'on veut mesurer en per-opératoire la valeur de l'angle A de correction angulaire nécessaire sur l'os, on peut utiliser un rapporteur tel qu'illustré sur les figures 10 et 11. Ce rapporteur comprend un corps 47 solidaire d'un manche 48 et comportant une tige 49 que l'on vient introduire dans l'un des tunnels précédemment forés dans l'os à l'aide du guide de perçage représenté sur les figures 7 à 9. Un palpeur 50 est articulé sur le corps 47 selon un axe transversal 51. On applique le corps 47 sur la partie proximale d'os après ostéotomie, et l'on applique le palpeur 50 sur la partie distale d'os en réglant l'inclinaison de la partie distale d'os pour obtenir la correction désirée. On peut alors lire l'angle formé entre le corps 47 et le palpeur 50 sur une graduation 151 solidaire du corps et devant laquelle se déplace une aiguille 52 solidaire du palpeur 50.

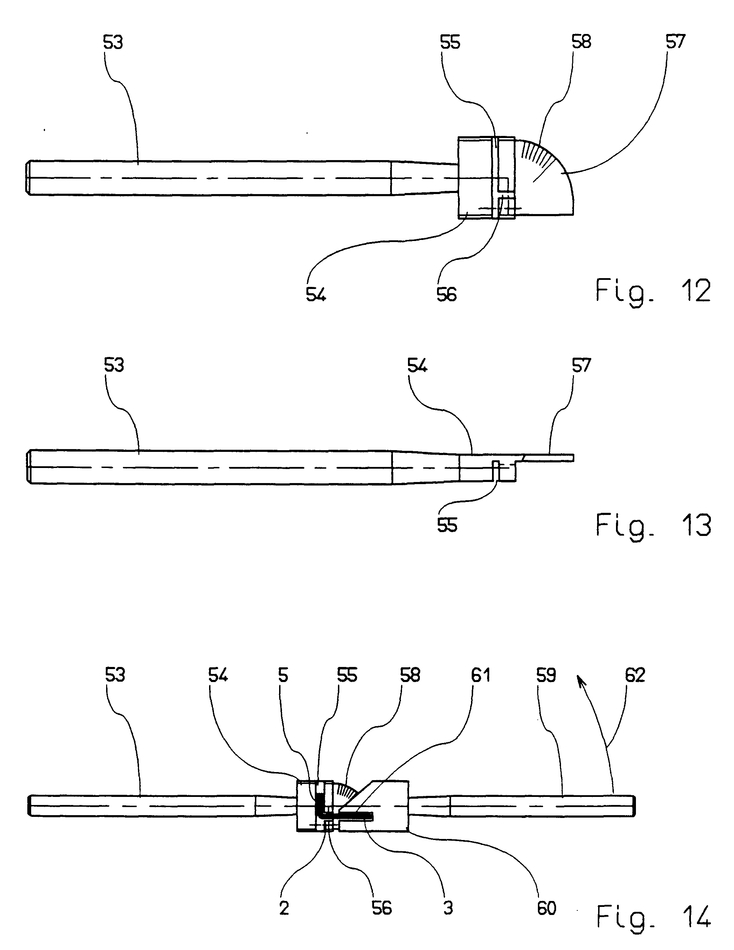

Un dispositif cintreur est utilisé pour conformer le corps 1 selon l'angle A choisi. Le dispositif cintreur comprend un premier élément, illustré sur les figures 12 et 13, muni d'un manche 53 et d'un corps 54. Le corps 54 comprend une grande rainure transversale 55 et une petite rainure longitudinale 56, conformées pour permettre l'introduction latérale de l'extrémité de corps de plaque-agrafe comportant les broches de premier élément d'ancrage 5. Les broches sont introduites dans la grande rainure transversale 55, tandis que l'extrémité du corps 1 traverse la petite rainure longitudinale 56. Un flasque latéral 57 portant une graduation angulaire 58 permet de mesurer l'angle A de raccordement angulaire du corps 1.

Pour régler cet angle A, on utilise un second élément comme illustré sur la figure 14, comprenant lui-même un manche 59 et un corps 60 muni d'une fente longitudinale 61. On introduit le second segment principal 3 du corps 1 longitudinalement dans la fente 61, en opposition avec le premier segment principal 2 de corps 1 introduit dans le premier élément illustré sur les figures 12 et 13. En manoeuvrant les manches 53 et 59 comme indiqué par la flèche 62, on peut cintrer le corps 1 selon l'angle A désiré, mesuré sur la graduation 58.

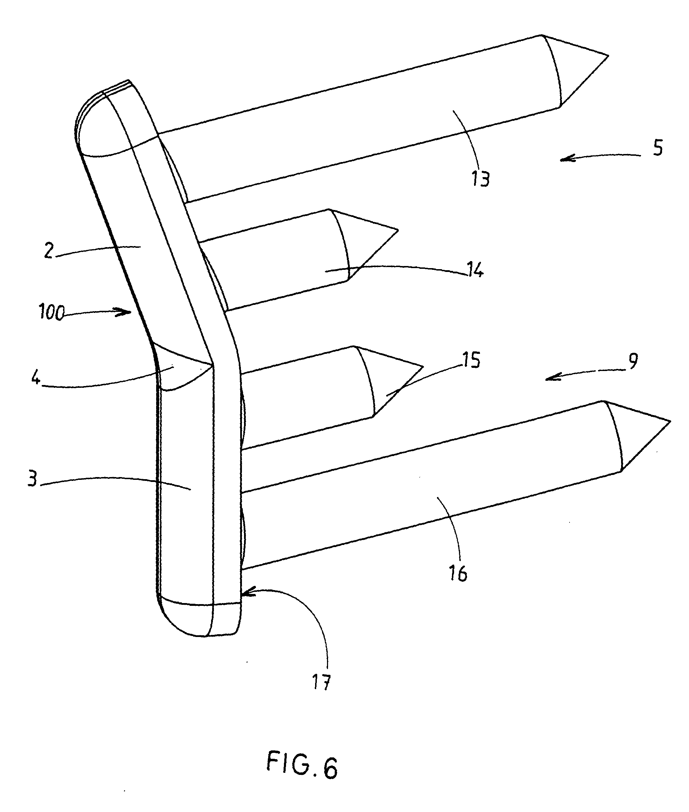

Sur la figure 6, on a représenté un dispositif complémentaire de l'invention, ayant un corps 100 allongé rigide, comportant un premier segment principal 2 rectiligne et un second segment principal 3 rectiligne se raccordant angulairement l'un à l'autre par une zone de raccordement angulaire 4 réalisant un angle sensiblement égal à la correction angulaire à réaliser sur un os, par exemple égal à 10° environ. Le premier segment principal 2 comprend des premiers moyens d'ancrage 5, et le second segment principal 3 comprend des seconds moyens d'ancrage 9.

Dans ce dispositif complémentaire, les premiers moyens d'ancrage 5 comprennent un premier groupe de deux broches 13 et 14, décalées longitudinalement sur le corps 100. La broche d'extrémité 13 est plus longue que la broche intermédiaire 14.

De même, les seconds moyens d'ancrage 9 comprennent un second groupe de deux broches 15 et 16, décalées longitudinalement sur le corps 100, la broche d'extrémité 16 étant plus longue que la broche intermédiaire 15.

Les broches 13, 14, 15 et 16 peuvent avantageusement être parallèles les unes aux autres, et font saillie sur la face convexe 17 du corps 100.

Le corps 100 présente une longueur avantageusement comprise entre le tiers et la moitié de la longueur de l'os à corriger. Dans le cas d'une correction de première phalange du premier rayon de pied, la longueur du corps 1 est avantageusement comprise entre 1 et 2 cm environ.

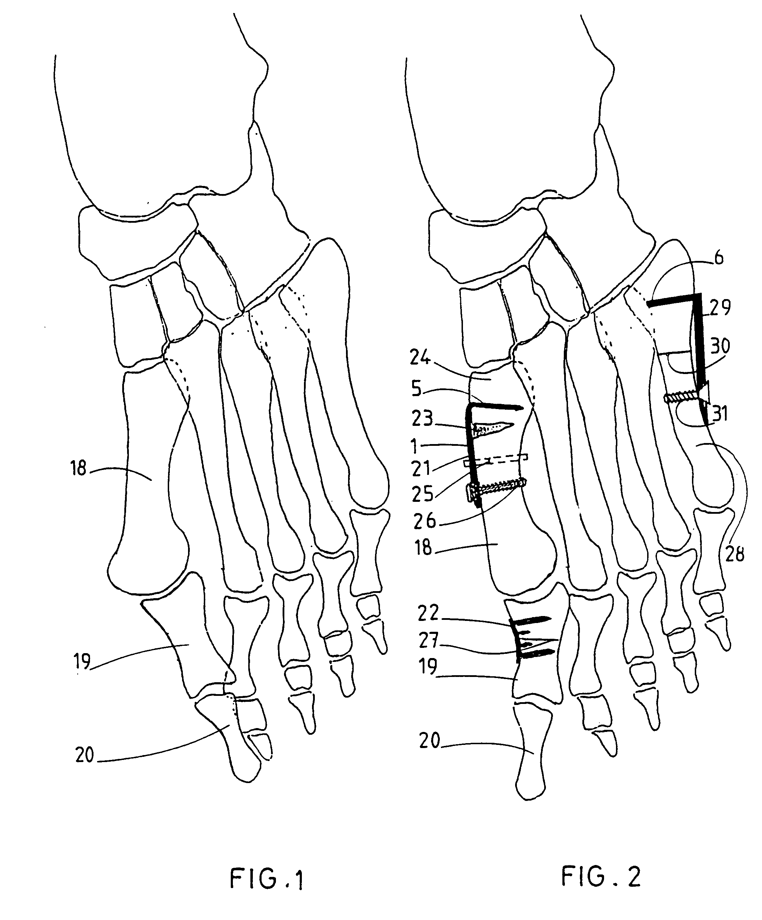

Comme illustré sur la figure 2, le matériel utilisé pour la correction des déformations du pied, notamment pour un Hallux Valgus, comprend avantageusement un premier dispositif tel qu'une plaque-agrafe 21 selon le mode de réalisation des figures 3 à 5, conformée pour une adaptation sur le premier métatarsien 18 de premier rayon du pied, et un second dispositif complémentaire tel qu'une agrafe selon le mode de réalisation de dispositif complémentaire de la figure 6, conformée pour une adaptation sur la première phalange 19 adjacente au dit premier métatarsien 18.

La figure 1 illustre un pied présentant un Hallux Valgus à corriger : le premier rayon du pied est formé par le premier métatarsien 18, la première phalange 19 et la seconde phalange 20, réalisant un flambage du premier rayon du pied entraînant une saillie angulaire interne de la métatarso phalangienne.

Sur la figure 2, on a illustré le même pied après correction par le matériel selon l'invention : le premier métatarsien 18 est corrigé par une plaque-agrafe 21 selon le mode de réalisation des figures 3 à 5 de l'invention, tandis que la première phalange 19 est corrigée par une agrafe 22 selon le mode de réalisation de dispositif complémentaire de la figure 6 de l'invention.

Les temps opératoires sont les suivants :
- dans une zone intermédiaire du premier métatarsien 18, on réalise à la scie une ostéotomie épiphyso-diaphysaire 23 d'ouverture externe, respectant la corticale externe, en prévoyant un apport osseux angulaire d'angle égal à la correction à obtenir ;
- au moyen du guide de perçage adapté, on fore deux tunnels parallèles entre eux dans la partie proximale 24 spongieuse épiphysaire du premier métatarsien 18, les tunnels ayant une direction faisant un angle B avec l'axe longitudinal du métatarsien, les tunnels étant positionnés de façon que la zone de raccordement angulaire 4 de la plaque-agrafe 21 soit sensiblement en regard de l'ostéotomie 23 ;
- on place la plaque-agrafe 21 en insérant les broches du premier moyen d'ancrage 5 dans les tunnels forés dans la partie proximale 24 du premier métatarsien 18, le corps 1 de la plaque-agrafe 21 étant préalablement angulé à l'aide du dispositif cintreur pour obtenir la correction souhaitée telle que préalablement mesurée à partir d'une radio du pied en charge, et/ou en per-opératoire à l'aide du rapporteur appliqué sur l'os après ostéotomie ;
- on immobilise provisoirement la plaque-agrafe 21 par une broche diaphysaire 25 introduite par le trou 12 approprié tel qu'illustré sur les figures 3 à 5 ;
- on vérifie le bon alignement osseux dans tous les plans ;
- on fixe définitivement la plaque-agrafe 21 par une vis à os 26 dans la diaphyse du premier métatarsien 18 ;
- on retire finalement la broche diaphysaire 25, que l'on peut toutefois laisser en place en cas de montage instable.

Si la correction d'un Hallux Valgus ainsi pratiquée n'est pas suffisante, il faut agir également sur la première phalange 19 par une ostéotomie de fermeture interne diaphysaire 27. L'angulation de la soustraction osseuse est déterminée par un guide de coupe dont l'angle peut être par exemple de 5°, 10° ou 15° environ.

La fermeture est stabilisée par l'agrafe 22 selon le mode de réalisation de dispositif complémentaire de la figure 6, constituant une agrafe à quatre broches d'inégales longueurs, deux broches allant dans chaque fragment osseux pour éviter tout phénomène de bielle.

Cette agrafe 22 est mise en place après un préperçage osseux grâce à un ancillaire adapté. L'ancillaire et l'agrafe 22 présentent la particularité d'avoir une angulation appropriée qui leur permet d'épouser la forme de la diaphyse après fermeture osseuse lors de leur application.

Le préperçage est positionné de façon que l'agrafe se place avec sa zone de raccordement angulaire 4 sensiblement en regard de l'ostéotomie 27.

On comprendra que l'agrafe 22 peut être utilisée indépendamment de l'usage d'une plaque-agrafe 21.

Sur la figure 2, on a également illustré la correction de rotation du cinquième métatarsien 28, par une plaque-agrafe 29 à une broche selon le mode de réalisation de la figure 5. Après ostéotomie intermédiaire 30 et correction en rotation axiale des deux tronçons de cinquième métatarsien 28 l'un par rapport à l'autre, la plaque-agrafe 29 est ancrée par sa broche unique 6 dans le premier segment, et fixée au second segment par une vis à os 31.

La présente invention n'est pas limitée aux modes de réalisation qui ont été explicitement décrits, mais elle en inclut les diverses variantes et généralisations contenues dans le domaine des revendications ci-après.

## Revendications

1. Matériel pour la correction de déformations osseuses des petits os longs du corps humain, comprenant au moins un dispositif (21) de maintien de l'os comportant :
- un corps (1) allongé rigide comportant deux segments principaux (2, 3) rectilignes solidaires,
- des premiers moyens d'ancrage (5), prévus sur le premier segment principal (2) du corps (1) pour se fixer transversalement dans un os, les premiers moyens d'ancrage (5) comprenant au moins une broche (6, 7) se développant selon une direction faisant généralement un angle déterminé (B) par rapport à l'axe longitudinal du premier segment principal (2) de corps (1),
- des seconds moyens d'ancrage (9), prévus sur le second segment principal (3) du corps (1) et adaptés pour se fixer transversalement dans l'os au cours d'une étape ultérieure, après fixation des premiers moyens d'ancrage (5),
caractérisé en ce que :
- les deux segments principaux se raccordent angulairement l'un à l'autre par une zone de raccordement angulaire (4) selon un angle (A) adaptable pour être sensiblement égal à la correction angulaire à réaliser sur l'os,
- ledit corps (1) est en un matériau et selon une forme de plaque admettant sans rupture et conservant les déformations angulaires permanentes dans la zone de raccordement angulaire (4),
- la zone de raccordement angulaire a une épaisseur (E) sensiblement inférieure à sa largeur (L1),
de sorte que le matériel est adapté pour être angulé à l'aide d'un cintreur pour obtenir la correction souhaitée.

2. Matériel selon la revendication 1, caractérisé en ce que :
- les seconds moyens d'ancrage (9) comprennent au moins un trou (10) d'adaptation de vis à os,
- les moyens d'ancrage (5, 9) sont adaptés pour pénétrer dans l'os en faisant saillie sur la face concave (8) du corps (1).

3. Matériel selon la revendication 2, caractérisé en ce que le corps (1) comprend, au voisinage du trou (10) d'adaptation de vis à os, un trou secondaire (12).

4. Matériel selon l'une quelconque des revendications 1 à 3, caractérisé en ce que l'angle (A) formé entre les deux segments principaux (2, 3) du corps (1) est égal à 15° environ.

5. Matériel selon l'une quelconque des revendications 1 à 4, caractérisé en ce que le corps (1) présente une longueur (L2) comprise entre le tiers et la moitié de la longueur de l'os qu'il est destiné à corriger.

6. Matériel selon l'une quelconque des revendications 1 à 5, caractérisé en ce qu'il comprend un guide de perçage adapté pour forer des trous de pénétration des premiers moyens d'ancrage (5) selon une direction faisant un angle (B) choisi avec l'axe longitudinal de l'os, et un dispositif cintreur adapté pour conformer le corps (1) du premier dispositif (21) de maintien de l'os selon l'angle (A) choisi.

7. Matériel selon l'une quelconque des revendications 1 à 6, caractérisé en ce qu'il comprend un dispositif complémentaire ayant un corps (100) allongé rigide composé d'un premier segment principal (2) rectiligne et d'un second segment principal (3) rectiligne se raccordant l'un à l'autre par une zone de raccordement angulaire (4), et dans lequel :
- des premiers moyens d'ancrage (5) comprennent un premier groupe de deux broches (13, 14) décalées longitudinalement sur le corps (100),
- des seconds moyens d'ancrage (9) comprennent un second groupe de deux broches (15, 16) décalées longitudinalement sur le corps (100),
- les broches (13-16) sont parallèles les unes aux autres et font saillie sur la face convexe (17) du corps (100).

8. Matériel selon la revendication 7, caractérisé en ce que les broches (13, 14 ; 15, 16) d'un même groupe de moyens d'ancrage sont de longueurs inégales, avec une broche d'extrémité (13, 16) plus longue que la broche intermédiaire (14, 15).

9. Matériel selon l'une des revendications 7 ou 8, caractérisé en ce que l'angle formé entre les deux segments principaux (2, 3) de corps (100) est égal à 10° environ.

10. Matériel pour la correction de déformations osseuses du pied, caractérisé en ce qu'il comprend :
- un premier dispositif (21) selon l'une quelconque des revendications 1 à 5, conformé pour une adaptation sur le premier métatarsien (18) de premier rayon du pied,
- un second dispositif complémentaire (22) selon l'une quelconque des revendications 7 à 9, conformé pour une adaptation sur la première phalange (19) adjacente au dit premier métatarsien (18).

## Patentansprüche

1. Gerät zur Korrektur von Knochendeformitäten der kleinen langen Knochen des menschlichen Körpers, welches wenigstens eine Vorrichtung (21) zur Abstützung des Knochens aufweist, die umfaßt:
- einen starren, länglichen Körper (1) mit zwei geraden, einstückig miteinander ausgebildeten Hauptabschnitten (2, 3),
- erste Verankerungsmittel (5), die am ersten Hauptabschnitt (2) des Körpers (1) so vorgesehen sind, daß sie sich in Querrichtung in einem Knochen festsetzen, wobei die Verankerungsmittel (5) wenigstens einen Dorn (6, 7) aufweisen, der sich in einer Richtung erstreckt, die mit der Längsachse des ersten Hauptabschnittes (2) des Körpers (1) einen bestimmten Winkel (B) bildet,
- zweite Verankerungsmittel (9), die am zweiten Hauptabschnitt (3) des Körpers (1) vorgesehen und so ausgebildet sind, daß sie nach der Fixierung der ersten Verankerungsmittel (5) im Verlauf einer weiteren Phase eine Fixierung im Knochen in Querrichtung herstellen,
dadurch gekennzeichnet, daß
- die beiden Hauptabschnitte durch einen winkelförmigen Verbindungsbereich (4) entsprechend einem Winkel (A) winklig miteinander verbunden sind, wobei dieser Winkel (A) im wesentlichen übereinstimmend mit der am Knochen herzustellenden Winkelkorrektur einstellbar ist,
- der Körper (1) aus einem Werkstoff und in einer Plattenform besteht, die permanente Winkelverformungen in dem winkelförmigen Verbindungsbereich (4) bruchfrei und konservierend gestatten,
- der winkelförmige Verbindungsbereich eine Dicke (E) hat, die deutlich kleiner als seine Breite (L1) ist,
so daß das Gerät mittels eines Biegewerkzeugs zur Erzeugung der gewünschten Korrektur abgewinkelt werden kann.

2. Gerät nach Anspruch 1, dadurch gekennzeichnet,
- daß die zweiten Verankerungsmittel (9) wenigstens ein Loch (10) zur Aufnahme einer Knochenschraube haben
- und daß die Verankerungsmittel (5, 9) so ausgebildet sind, daß sie, abstehend von der konkaven Seite (8) des Körpers (1), in den Knochen eingreifen.

3. Gerät nach Anspruch 2, dadurch gekennzeichnet, daß der Körper (1) in der Nähe des Loches (10) zur Aufnahme der Knochenschraube ein Sekundärloch (12) aufweist.

4. Gerät nach einem der Ansprüche 1 bis 3, dadurch gekennzeichnet, daß der Winkel (A) zwischen den beiden Hauptabschnitten (2, 3) des Körpers (1) etwa 15° beträgt.

5. Gerät nach einem der Ansprüche 1 bis 4, dadurch gekennzeichnet, daß die Länge (L2) des Körpers (1) zwischen einem Drittel und der Hälfte der Länge des zu korrigierenden Knochens beträgt.

6. Gerät nach einem der Ansprüche 1 bis 5, dadurch gekennzeichnet, daß dieses auch eine Bohrungsführung zum Bohren der Löcher für die Aufnahme der ersten Verankerungsmittel (5) in einer Richtung, welche mit der Längsachse des Knochens einen ausgewählten Winkel (B) bildet, sowie ein Biegewerkzeug aufweist, mit dessen Hilfe der Körper (1) der ersten Vorrichtung (21) zur Abstützung des Knochens entsprechend dem gewählten Winkel (A) verformt werden kann.

7. Gerät nach einem der Ansprüche 1 bis 6, dadurch gekennzeichnet, daß dieses auch ein zusätzliches Gerät aufweist, das einen starren, länglichen Körper (100) mit einem ersten geraden Hauptabschnitt (2) und einem zweiten geraden Hauptabschnitt (3) hat, die miteinander über einen winkelförmigen Verbindungsbereich (4) verbunden sind, wobei in dem zusätzlichen Gerät
- erste Verankerungsmittel (5) eine erste Gruppe von zwei auf dem Körper (100) in Längsrichtung zueinander versetzten Dornen (13, 14) haben,
- zweite Verankerungsmittel (9) eine zweite Gruppe von zwei auf dem Körper (100) in Längsrichtung zueinander versetzten Dornen (15, 16) haben,
- wobei die Dorne (13 - 16) zueinander parallel verlaufen und von der konvexen Seite (17) des Körpers (100) abstehen.

8. Gerät nach Anspruch 7, dadurch gekennzeichnet, daß die Dorne (13, 14; 15, 16), jeweils einer Gruppe von Verankerungsmitteln unterschiedliche Längen haben, wobei ein außenstehender Dorn (13, 16) länger als der dazwischenstehende Dorn (14, 15) ist.

9. Gerät nach Anspruch 7 oder 8, dadurch gekennzeichnet, daß der Winkel, der von den beiden Hauptabschnitten (2, 3) des Körpers (100) gebildet wird, etwa 10° beträgt.

10. Gerät zur Korrektur von Knochendeformitäten des Fußes, dadurch gekennzeichnet, daß dieses umfaßt
- eine erste Vorrichtung (21) nach einem der Ansprüche 1 bis 5, das zur Anpassung an den ersten Metatarsus (18) des ersten Fußstrahles ausgebildet ist,
- eine zweite, zusätzliche Vorrichtung (22) nach einem der Ansprüche 7 bis 9, die zur Anpassung an die erste Phalanx (19) ausgebildet ist, die sich an den ersten Metatarsus (18) anschließt.

## Claims

1. Equipment for correcting deformation of small long bones in the human body, comprising at least one bone holding device (21) including:
- a rigid elongate body (1) having two rectilinear main segments (2, 3) joined together,
- first anchor means (5) on the first main segment (2) of the body (1) adapted to be fixed transversely into a bone, the first anchor means (5) including at least one pin (6, 7) extending in a direction substantially at a particular angle (B) relative to the longitudinal axis of the first main segment (2) of the body (1),
- second anchor means (9) on the second main segment (3) of the body (1) adapted to be fixed transversely in the bone at a subsequent stage, after fixing of the first anchor means (5),
characterized in that :
- the two main segments are joined together angularly by an angled joining area (4) at an angle (A) which can be varied so that it is substantially equal to the required angular correction to be applied to the bone,
- said body (1) is made from a material and has a plate shape adapted to withstand without breaking and to retain permanent angular deformation in the angled joining area (4),
- the angled joining area has a thickness (E) substantially smaller than its width (L1),
so that the equipment is adapted to be bent by means of a bending device to obtain the required correction.

2. Equipment according to claim 1, characterized in that:
- the second anchor means (9) include at least one hole (10) adapted to fit a bone screw,
- the anchor means (5, 9) are adapted to penetrate the bone and project from the concave face (8) of the body (1).

3. Equipment according to claim 2, characterized in that the body (1) includes, near the hole (10) adapted to fit a bone screw, a secondary hole (12).

4. Equipment according to any one of claims 1 to 3, characterized in that the angle (A) between the two main segments (2, 3) of the body (1) is equal to approximately 15°.

5. Equipment according to any one of claims 1 to 4, characterized in that the body (1) has a length (L2) between one third and one half the length of the bone which it is to correct.

6. Equipment according to any one of claims 1 to 5, characterized in that it includes a drilling guide for boring penetration holes for the first anchor means (5) in a direction at a particular angle (B) to the longitudinal axis of the bone, and a bending device adapted to shape the body (1) of the first bone holding device (21) to the required angle (A).

7. Equipment according to any one of claims 1 to 6, characterized in that it includes a complementary device having a rigid elongate body (100) comprising a rectilinear first main segment (2) and a rectilinear second main segment (3) joined together at an angled joining area (4), and wherein:
- first anchor means (5) include a first pair of pins (13, 14) offset longitudinally on the body (100),
- second anchor means (9) include a second pair of pins (15, 16) offset longitudinally on the body (100),
- the pins (13-16) are parallel to each other and project from the convex side (17) of the body (100).

8. Equipment according to claim 7, characterized in that the pins (13, 14; 15, 16) of the same pair of anchor means are of different lengths, with an end pin (13, 16) longer than the intermediate pin (14, 15).

9. Equipment according to claim 7 or claim 8,
characterized in that the angle between the two main segments (2, 3) of the body (100) is equal to approximately 10°.

10. Equipment for correcting deformation of bones of the foot, characterized in that it includes:
- a first device (21) according to any one of claims 1 to 5, shaped to fit to the first metatarsal (18) of the first hallux,
- a complementary second device (22) according to any one of claims 7 to 9, shaped to fit to the first phalange (19) adjacent said first metatarsal (18).
